# EUROPÄISCHE PATENTANMELDUNG

(11) **EP 0 794 247 A1**
(43) Veröffentlichungstag der Anmeldung: **10.09.1997**
(21) Anmeldenummer: 97810124.4
(22) Anmeldetag: 05.03.1997
(51) Int. Cl.: C12M 1/113

(54) **Fermenter**

(30) Priorität: 05.03.1996 CH 567/96
(71) Anmelder: Rindelaub, Frank Alex Erich, 2014 Bôle (CH)
(72) Erfinder: Rindelaub, Frank Alex Erich, 2014 Bôle (CH)
(74) Vertreter: Braun, André, jr.

(57) **Zusammenfassung**

Der Fermenter besitzt eine abwechselnd in beide Drehrichtungen rotierende Trommel (4). Die Trommel ist mit einer eingebauten Spirale (17) versehen, die den Innenraum in einen durchgängigen spiraligen Kanal unterteilt. Das zu vergärende Material durchläuft diesen Kanal nach dem Prinzip des Propfenstroms. Die Verweilzeit wird durch die Länge des Kanals und durch die Kombination aus Vorwärts- und Rückwärtsdrehungen der Trommel bestimmt. Zwischen den Wendeln der Spirale (17) sind achsparallele Rührschaufeln (19) angeordnet.

## Beschreibung

Die Erfindung betrifft einen rotierenden Fermenter. Unter Fermenter wird in dieser Beschreibung in Übereinstimmung mit der fachüblichen Terminologie eine Anlage zur Fermentation von biologisch abbaubaren Ausgangsstoffen verstanden. Dabei steht die anaerobe Behandlung von Müll im Vordergrund. Bei der anaeroben Fermentation entsteht Biogas.

Rotierende Fermenter sind bekannt. Beispielsweise ist in WO-A-86/05171 ein Fermenter für organisches Material beschrieben, das mit einem Anteil an biologisch nicht abbaubaren Stoffen, wie Sand, Kies, Metallstücke, usw., vermischt ist. Diese sollen schneller aus der Anlage entfernt werden als die biologisch abbaubaren Stoffe, die eine längere Verweilzeit brauchen. Zu diesem Zweck besitzt der rotierende Fermentationsbehälter Führungsrippen (37-39) entlang der Behälterwand und entlang der eingebauten achsparallelen Rührflügel. Der Innenraum des Reaktionsbehälters ist außer den Rührflügeln durch eine achssenkrechte Trennwand unterteilt.
In DE-A-3325257 ist ein Fermenter mit einem rotierenden Trommelreaktor zur Gewinnung von Biogas beschrieben. Auch dieser Reaktor hat einen großen offenen Innenraum, der ebenfalls einige achssenkrechte Trennwände besitzt.

Es hat sich gezeigt, daß bekannte rotierende Fermenter eine Reihe von Nachteilen aufweisen. So ist zum Beispiel die Verweilzeit des abbaufähigen Materials im Fermenter nicht präzis kontrollierbar. Für frisch zugeführtes Material hängt es von zufälligen Bedingungen ab, ob es nach längerer oder kürzerer Verweilzeit an der Entnahmeöffnung ankommt. Ein streng nach der Reihenfolge der Zuführung kontrollierter Durchsatz ist in den Reaktoren mit einem offenen Innenraum nicht möglich. Ein weiterer Nachteil sind quer zur Materialbewegung angeordnete Einbauten, zum Beispiel Schikanen, Rührer, usw. . Vor allem bei der Verarbeitung von Restmüll, d. h. Müll, der nach dem Aussortieren von rezyklierbaren Stoffen übrigbleibt, tritt eine sehr hohe mechanische Belastung durch Sedimente, d. h. schwere, biologisch nicht abbaubare Feststoffe, wie Steine, Metallteile, Glas, auf. Dies führt dazu, daß quer angeordnete Einbauten in kurzer Zeit beschädigt oder sogar zerstört werden, so daß eine Reparatur erforderlich wird. Dies ist nicht vereinbar mit der üblichen Forderung, daß eine derartige Anlage mehrere Jahre wartungsfrei laufen muß.

Der Erfindung liegt die Aufgabe zugrunde, einen rotierenden Fermenter vorzuschlagen, der von den vorgenannten Nachteilen frei ist.
Erfindungsgemäß wird diese Aufgabe gelöst, durch einen rotierenden Fermenter mit den in den Patentansprüchen definierten Merkmalen.

Im folgenden ist anhand der beiliegenden Zeichnungen eine bevorzugte Ausführungsform der Erfindung beschrieben.

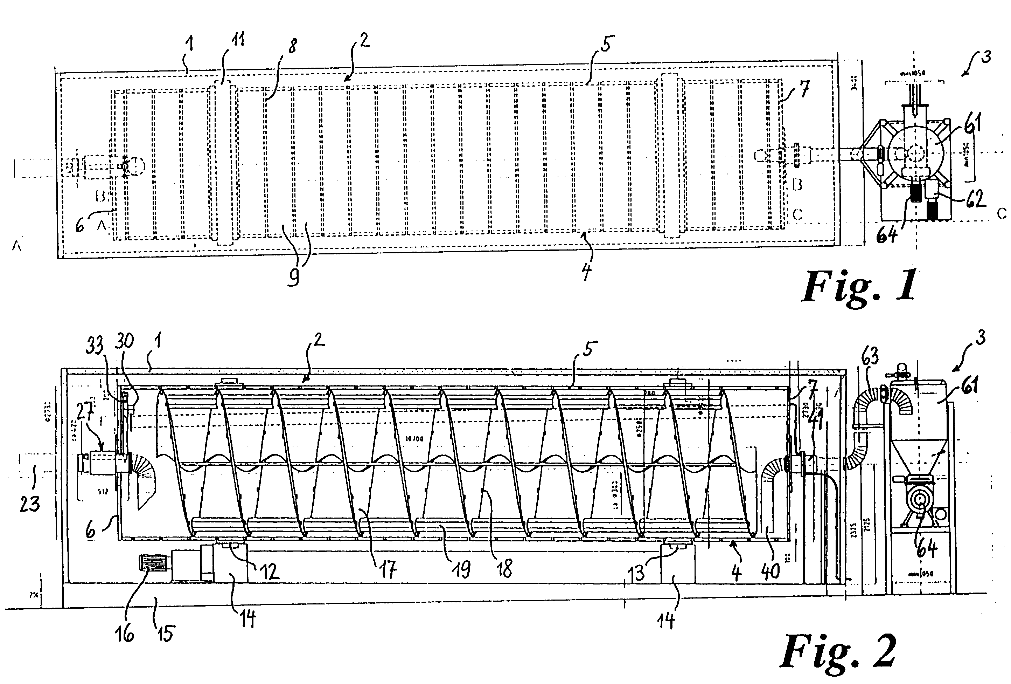

Es zeigen :
- Fig. 1: eine Draufsicht auf eine Fermentationsanlage,
- Fig. 2: einen Längsschnitt durch die Anlage entlang der Linie A-A-B-B-C-C der Fig. 1,
- Fig. 3: eine Seitenansicht der Einbauten,
- Fig. 4: einen Schnitt durch eine Rührschaufel,
- Fig. 5: einen Querschnitt durch die Einbauten entlang A-A in Fig. 3,
- Fig. 6: einen Schnitt durch Lager und Durchführung der Beschickungsrohres,
- Fig. 7: einen Schnitt durch Lager und Durchführung des Entnahmerohres.
Wie die Draufsicht in Fig. 1 und die Schnittdarstellung in Fig. 2 zeigen, ist die Anlage von einem geschlossenen äußeren Behälter 1 umgeben, in dem ein drehbarer Fermenter 2 angeordnet ist. Außerhalb des Behälters 1 befindet sich lediglich eine Austragseinrichtung 3 zur Entnahme von Sinkstoffen und Fermentationsprodukten. Der Behälter 1 besteht aus isolierenden Elementen oder einem anderen Material, das nicht nur dem Witterungsschutz, sondern vor allem auch der Wärmedämmung dient. Alternativ könnte die Wärmeisolation auch direkt am Fermenter selbst angebracht sein. Der äußere Behälter würde sich dann erübrigen.

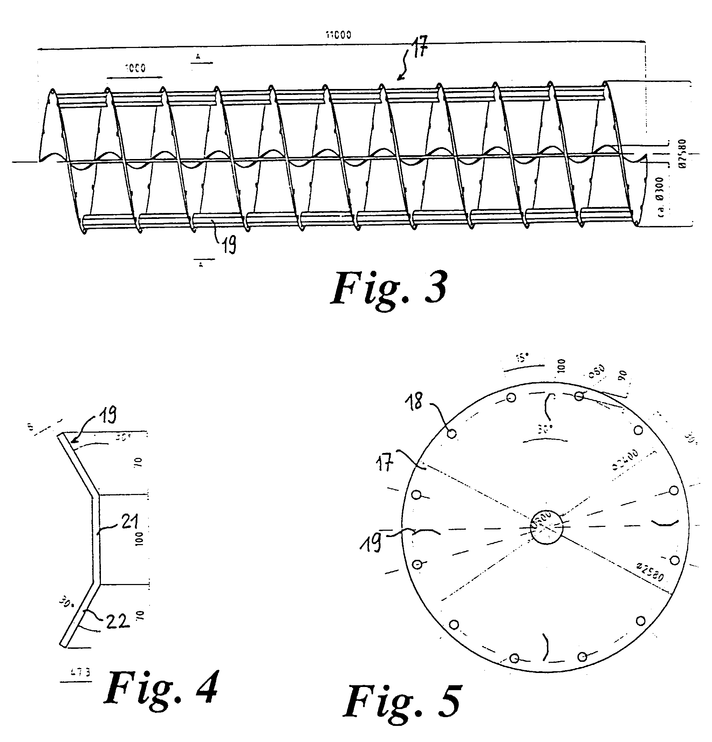

Der Fermenter 2 ist eine Trommel 4 aus doppelwandigem Rohr 5, das an seinen beiden Enden mit Stirnwänden 6-7 verschlossen ist. Der Zwischenraum zwischen den beiden Schalen des Doppelmantelrohres 5 ist durch Stege 8 in einzelne, das Rohr ringförmig umgebende Felder oder Taschen 9 unterteilt, die gruppenweise untereinander verbunden sind. Der Zwischenraum des Doppelmantelrohres wird mit Heizflüssigkeit beschickt. Durch die gruppenweise Aufteilung der Felder können in verschiedenen Bereichen des Fermenters unterschiedliche Heizleistungen vorgesehen werden. So kann zum Beispiel im Zuführungsbereich, in dem die Beschickung mit kaltem Material erfolgt, eine stärkere Heizleistung gebraucht werden. Die Verteilung der Heizmittelströme auf die einzelnen Felder und Bereiche erfolgt in an sich bekannter Weise mittels (nicht gezeigten) Ventilen. Die Trommel 4 ist auf ihrer Außenseite mit zwei umlaufenden Schienen 11 versehen, die auf zwei Rollenpaaren 12-13 gelagert sind. Die Lagerrollen 12-13 befinden sich auf Sockeln 14, die ihrerseits auf einem Fundament 15 der gesamten Anlage befestigt sind. Je nach Auslegung der Trommel kann es sinnvoll sein, mehr als zwei solche Lager vorzusehen. Das Fundament ist in Längsrichtung der Trommel 4 geringfügig geneigt. Die Neigung beträgt beim vorliegenden Beispiel 2%. Je nach Situation kann dieser Wert aber variieren. Die höhere Seite ist die Beschickungsseite, die niedrigere die Entnahmeseite der Anlage.
Das bei der Beschickungsseite befindliche Rollenpaar 12 ist durch einen Elektro-Getriebemotor 16 und Kette angetrieben, und dreht die Trommel 4. Der Antrieb erfolgt abwechselnd, entsprechend einem vorgegebenen oder durch Regelparameter veränderlichen Programm in beide Drehrichtungen. Die Einbauten der Trommel 4 sind in Fig. 2 und vor allem in den Fig. 3-4 gezeigt. Über den größten Teil der Längenausdehnung des Trommelinnenraumes erstreckt sich eine fest eingeschweißte Spirale 17, die den Innenraum in einen durchgehenden spiraligen Kanal unterteilt, den das Material während des Fermentationsprozesses durchlaufen muß. Zwischen den einzelnen Windungen dieses Kanals ergibt sich lediglich in Achsnähe ein geringfügiger, aber vernachlässigbarer Kurzschluß. Der Kanal wird also nach dem Prinzip des Propfenstroms, d. h. in der Reihenfolge der Materialbeschickung durchlaufen. Bei einem Trommeldurchmesser von ca. 3 m und einer Spirale mit zehn Windungen ergibt sich eine mittlere Kanallänge von nahezu 50 m. Diese Länge sichert eine Mindestverweilzeit des Materials im Fermenter.
Die Spirale 17 besteht aus dickwandigem verschleißfestem Blech. Dadurch, daß sie parallel zum Materialstrom angeordnet ist, treten keine extremen mechanischen Belastungen durch Steine, usw. auf.
Die Spirale ist nahe dem Außendurchmesser mit Löchern 18 versehen, die dem entstehenden Biogas den Durchtritt ermöglichen.
Die Wendeln der Spirale 17 sind durch achsparallel angeordnete Rührschaufeln 19 untereinander verbunden. Die Rührschaufeln haben die Funktion, die Bildung von Sink- und Schwimmschichten zu verhindern. Außerdem haben sie die Aufgabe, die Freisetzung des sich in der Masse bildenden Biogases zu fördern.
Die Form der Rührschaufeln 19 ist am besten aus Fig. 4, ihre Anordnung aus Fig. 5 ersichtlich. Sie besitzen einen flachen Mittelteil 21, der in radialer Richtung angeordnet ist, sowie zwei seitliche, gegen den Mittelteil in gleicher Richtung abgewinkelte Seitenteile 22. Dadurch ist auf der Vorderseite der Schaufeln ein bestimmtes Volumen definiert. Damit nehmen die Schaufeln 19 beim Drehen der Trommel 4 aus dem tiefliegenden Bereich Sinkstoffe auf und heben sie, bis sie etwa auf die Höhe des Füllniveaus der Gärmasse beginnen aus der Schaufel herauszugleiten und wieder nach unten abzusinken. Beim Weiterdrehen taucht die Schaufel auf der anderen Seite wieder in die Masse ein und nimmt an der Oberfläche angesammelte Schwimmstoffe mit nach unten, um sie erst nahe dem tiefsten Punkt wieder freizugeben. Dadurch werden die Schwimmstoffe ständig mit der Masse vermischt, so daß sich keine Schwimmschicht ausbilden kann.
Die Winkelstellung, an welcher die Sinkstoffe in die Masse zurückfallen bzw. die Schwimmstoffe freigegeben werden, hängt vom Winkel zwischen dem Mittelteil 21 und dem zur Trommelachse gerichteten Seitenteil 22 ab. Es kann zweckmäßig sein, diesen Winkel kleiner zu machen als den des äußeren Seitenteils.
Der Einbau der Schaufeln ist so vorgenommen, daß sie in Bezug auf eine Drehrichtung abwechselnd die Vorder- und die Rückseite vorne haben. Dadurch sind die einen Schaufeln vor allem für die Materialbewegung in eine
Drehrichtung, die anderen für die Materialbewegung in die andere Drehrichtung zuständig. Es ist aber auch möglich, daß weniger Schaufeln rückwärts gerichtet sind als vorwärts, oder daß alle Schaufeln in Vorwärtsrichtung zeigen.
Um Beschädigungen der Schaufeln 19 zu verringern oder zu vermeiden, sind sie aus verschleißfestem Stahl mit hoher Wandstärke gefertigt. Zusätzlich können die exponierten Kanten der Seitenteile 22 mit Hartmetall bestückt sein.

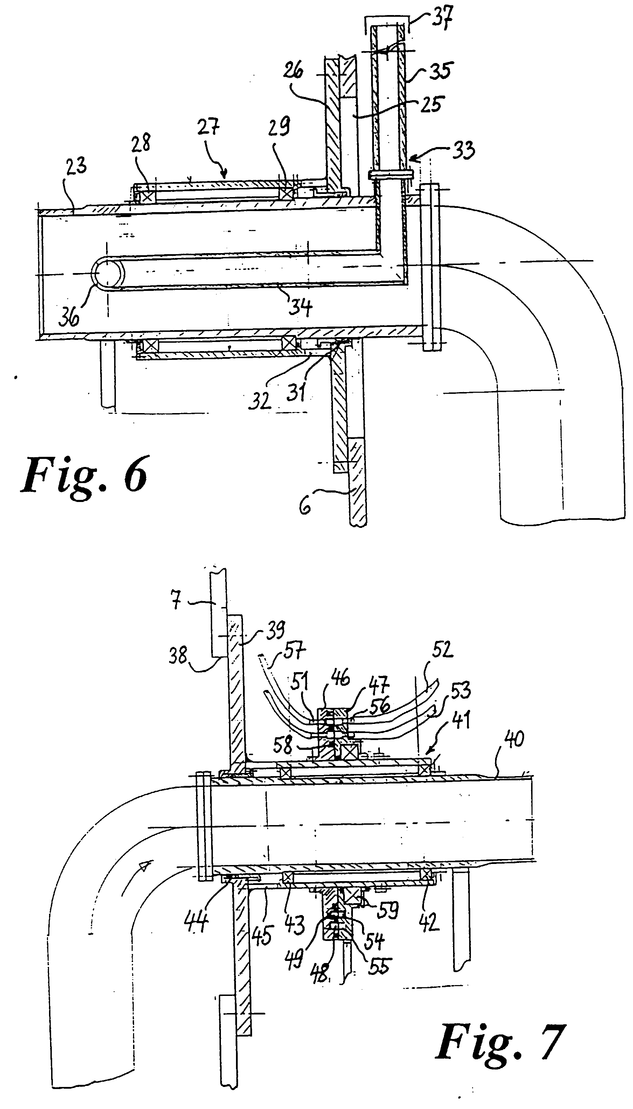

Auf der Beschickungsseite führt ein feststehendes Beschickungsrohr 23 durch die dortige Stirnwand 6 der Trommel 4 hindurch. Das Beschickungsrohr ist im Inneren der Trommel nach unten gebogen, damit der Materialeintritt etwa in der Mitte zwischen der Trommelachse und der Wand erfolgt. Dadurch ist sichergestellt, daß frisch zugeführtes Material nicht durch das Kernloch der Spirale 17 gedrückt wird, sondern sich in den Propfendurchgang einfügt.
Die Durchführung des Beschickungsrohres ist in Fig. 6 im Detail gezeigt. Die Stirnwand 6 hat eine Mittelöffnung 25, über der eine flache Deckplatte 26 und ein zylindrisches Lager- und Dichtungsgehäuse 27 angebracht sind, die das Beschickungsrohr koaxial umgeben. Zwischen dem Gehäuse 27 und dem Beschickungsrohr sind zwei Rollenlager 28-29 angeordnet. In der eigentlichen Durchführung des Beschickungsrohres 23 durch die Deckplatte 26 ist eine Stopfbüchse 31 angeordnet. Die Packung ist mittels mehrerer hintereinander angeordneter Tellerfedern (nicht gezeigt) mit einer über lange Zeit relativ konstanten Federkraft belastet. Durch die Rollenlager 28-29 ist dafür gesorgt, daß praktisch keine Exzentrizität zwischen Trommel 4 und Beschickungsrohr 23 auftreten kann, was die Lebensdauer der Stopfbüchsenpackung erheblich erhöht.
Das Gehäuse 27 besitzt bei seiner Befestigung an der Deckplatte 26 Schlitze 32, durch die evtl. doch an der Stopfbüchse auftretende Flüssigkeit abfliessen kann, so daß sie nicht in das benachbarte Rollenlager 29 gelangt und dort Korrosion verursacht.

Auf der Beschickungsseite ist auch die Gasentnahmeleitung 33 zur Entnahme von Biogas angeordnet. Ein koaxial im Inneren des Beschickungsrohres 23 angeordnetes Rohrstück 34 ist im Inneren der Trommel 4 mit einem nach oben aus dem Beschickungsrohr heraus bis in den Kopfraum der Trommel führenden, senkrechten Rohrstück 35, und außerhalb des Dichtungsgehäuses 27 mit einem seitlich aus dem Beschickungsrohr 23 herausführenden, waagerechten Rohrstück 36 verbunden. Diese drei miteinander verbundenen Rohrstücke 34-35-36 bilden einen Gasschnorchel zur Entnahme von Biogas aus dem Kopfraum des Fermenters. Das senkrechte Rohrstück 25 ist an seiner oberen Mündung mit einer Haube 37 gegen das Eindringen von Material abgedeckt.
Am senkrechten Rohrstück 35 ist eine Füllstandsmesseinrichtung 30 angebracht.

Auf der Entnahmeseite ist in ähnlicher Weise wie auf der Beschickungsseite ein feststehendes Entnahmerohr 40 durch die dortige Stirnwand 7 der Trommel 4 hindurchgeführt. Das Entnahmerohr 40 führt im Inneren der Trommel bis auf einen Abstand von ca. 100 mm an die Trommelwand heran. Die untere Öffnung des Entnahmerohres 40 dient zur periodischen Absaugung von Material.

Die Durchführung des Entnahmerohres 40 ist in Fig. 7 im Detail gezeigt. Die Stirnwand 7 hat eine Mittelöffnung 38, über der eine flache Deckplatte 39 und ein zylindrisches Lager- und Dichtungsgehäuse 41 angebracht sind, die das Entnahmerohr 40 konzentrisch umgeben. Zwischen dem Gehäuse 41 und dem Entnahmerohr 40 sind zwei Rollenlager 42-43 angeordnet. In der Deckeldurchführung ist eine im wesentlichen gleiche Stopfbüchse 44 wie diejenige auf der Beschickungsseite angeordnet. Das Gehäuse 41 ist am Übergang zur Deckplatte 39 mit Schlitzen 45 zur Ableitung durchsickender Flüssigkeit versehen.
Außen am Gehäuse 41 ist ein Flansch 46 befestigt, dem ein feststehender Ring 47 gegenübersteht. Die einander zugewandten Flächen von Flansch 46 und Ring 47 sind plan bis auf eine Anzahl von konzentrischen umlaufenden Nuten. Der Ring besitzt zwei solche Nuten 4849, von denen an drei Stellen Bohrungen zur Rückseite führen, die mit Schlauchstutzen 51 versehen sind. An den Schlauchstutzen sind Anschlußleitungen für den Heizungsvorlauf 52 und den Heizungsrücklauf 53 angeschlossen. Den Nuten 48 und 49 im Ring 47 gegenüber besitzt die plane Fläche des Flansches zwei ebensolche Nuten 54-55, die über Bohrungen und Schlauchstutzen 56 mit Verbindungsleitungen 57 zum Wärmeaustauschsystem in der Trommelwand verbunden sind. Die beiden einander gegenüberliegenden Nutenpaare bilden Übergangs- und Verteilkanäle für das Heizmedium. Zusätzlich zu den beiden Nuten 54-55 besitzt die Stirnfläche des Flansches 46 drei weitere Ringnuten 58 von geringer Tiefe, in denen Dichtungsringe angeordnet sind, die die Verteilkanäle voneinander und nach außen abdichten. Um auch die mechanische Belastung dieser Dichtungen möglichst gering zu halten, ist zwischen dem Ring 47 und der Außenwand des Gehäuses 41 ein weiteres Rollenlager 59 angeordnet.

Wie bereits erwähnt, befindet sich die Austragseinrichtung 3 außerhalb des Behälters 1. Sie besitzt einen Saug-/Drucktank 61, der mit einem Kompressor 62 evakuiert oder mit Überdruck beaufschlagt werden kann. Das Entnahmerohr ist mit dem Tank 61 über flexible Kupplungen 63 verbunden. Unterhalb des Tankes ist eine Fördereinrichtung 64 zum Transport des Austrags zur weiteren Verarbeitung angeordnet.

Im Betrieb wird durch das Beschickungsrohr Material in die Trommel gepumpt. Die Trommel wird zum Rühren vor- und rückwärts gedreht, wobei die Vorwärtsdrehung, d. h. die Drehung, die das Material im wendelförmigen Kanal in Richtung Entnahmeseite fördert, häufiger oder zeitlich länger geschieht. Die Differenz ergibt sich aus der angestrebten Verweilzeit des Materials bis zum vordefinierten Vergärungsgrad. Gleichzeitig fördert die Spirale die Sedimente Richtung Entnahmeseite.
Auf der Entnahmeseite wird periodisch Material entnommen. Dadurch wird die gewünschte Menge ausgegorenes Material abgesaugt.
Die Absaugung über den Saugtank verhindert, daß vor allem Sedimente in eine Pumpe gelangen und diese schnell verschleissen können. Vor der Entnahme wird im Saugtank ein ausreichender Unterdruck erzeugt. Danach wird die Verbindung zwischen Tank 61 und Entnahmerohr 40 geöffnet, wodurch zunächst die Sedimente, und in weiteren Zyklen das ausgegorene Material, abgesaugt werden. Zur Weiterförderung des abgesaugten Austrags wird die Verbindung zwischen Tank 61 und Entnahmerohr 40 geschlossen, und die Verbindung zur Fördereinrichtung 64 geöffnet. Im Tank wird ein Überdruck erzeugt und dadurch das Material hinausgefördert. Wenn keine Entnahme erfolgt, ist die Verbindung zwischen Entnahmerohr , Tank und Fördereinrichtung ständig offen. Dadurch, daß der höchste Punkt der Verbindung zwischen Entnahmerohr und Tank höher liegt als das normale Füllniveau der Trommel, ist sichergestellt, daß im Normalbetrieb kein Material austritt. Im Fall von störfallmäßiger Überfüllung oder Überdruck kann aber Material über diese offene Verbindung und die Austragseinrichtung entweichen.

## Patentansprüche

1. Fermentationsanlage zur Verarbeitung von fermentierbarem Material, insbesondere von Restmüll, mit einer rotierenden Trommel und einem Antrieb zum Drehen der Trommel in beide Drehrichtungen, sowie Beschickungs- und Entnahmeleitungen, dadurch gekennzeichnet, daß im Inneren der Trommel (4) eine Spirale (17) angeordnet ist, die den Innenraum in einen durchgängigen spiraligen Kanal unterteilt, den das zu vergärende Material durchläuft.

2. Fermentationsanlage nach Anspruch 1, dadurch gekennzeichnet, daß zwischen den Wendeln der Spirale (17) achsparallelel Rührschaufeln (19) angeordnet sind.

3. Fermentationsanlage nach Anspruch 2, dadurch gekennzeichnet, daß die Kanten der Rührschaufeln (19) aus verschleißfestem Material bestehen.

4. Fermentationsanlage nach Anspruch 1, gekennzeichnet durch feststehende Beschickungs- und Entnahmerohre (23-40), die durch an ihnen rollengelagerte Durchführungen an den Stirnwänden (6-7) geführt sind.
